# EUROPÄISCHE PATENTSCHRIFT

(11) **EP 1 448 908 B1**
(45) Veröffentlichungstag und Bekanntmachung des Hinweises auf die Patenterteilung: **01.02.2006**
(21) Anmeldenummer: 02787769.5
(22) Anmeldetag: 22.11.2002
(51) Int. Cl.: F16D 3/48

(54) **Implantat**
Implant
Implant

(30) Priorität: 23.11.2001 DE 10157316; 23.11.2001 DE 10157315
(43) Veröffentlichungstag der Anmeldung: 25.08.2004
(73) Patentinhaber: Universität Duisburg-Essen, 45141 Essen (DE)
(72) Erfinder: WAGENER, Sven, 45219 Essen (DE); FISCHER, Alfons, 45239 Essen (DE)
(74) Vertreter: Gesthuysen, von Rohr & Eggert
(86) Internationale Anmeldenummer: PCT/EP2002/013123
(87) Internationale Veröffentlichungsnummer: WO 2003/044383

(56) Entgegenhaltungen:
- EP-A- 1 085 222
- DE-A- 2 944 052
- DE-A- 19 603 353
- US-A- 4 973 068
- US-A- 4 986 834
- US-A- 5 896 664

## Beschreibung

Die vorliegende Erfindung betrifft ein Implantat mit einem Lager und ein Verfahren zur Herstellung eines Implantats.

Besonders problematisch ist jeweils die Partikelbildung, die insbesondere bei einem Gleitlager zu einer unerwünschten sogenannten Drei-Körper-Abrasion (Lagerfläche/Partikel/Gegenlagerfläche) führen kann. Dies kann insbesondere in einer unerwünschten Bildung von Kratzern, Rillen o. dgl. in der Lagerfläche und der Gegenlagerfläche mit der Folge einer Verringerung der Standzeit resultieren. Weiter kann dies zu einer unerwünschten Schwergängigkeit bzw. Erhöhung der Reibung führen.

Bei einem Implantat mit einer Lagerfläche, insbesondere einem Hüftgelenk oder dergleichen, ist eine lange Standzeit bei geringer Reibung erwünscht. Häufig werden Lagerflächen aus Metall, Keramik oder Kunststoff eingesetzt. Besonders kritisch ist die genannte Partikelbildung, da sich lösende Partikel zu unerwünschten Nebenwirkungen in einem Körper mit dem implantierten Implantat führen können.

Die DE 29 44 052 A1 offenbart ein Gleitlagerelement mit einer durch Anätzen profilierten Lagermetallschicht. Auf die Lagermetallschicht ist eine Laufschicht auf Kunststoffbasis aufgebracht. Es findet sich jedoch kein Hinweis auf eine Anwendung in einem Implantat.

Die US 4,986,834 A offenbart ein Implantat, das insbesondere als Hüftgelenk einsetzbar ist und ein Kugelgelenk bildet.

Der vorliegenden Erfindung liegt die Aufgabe zugrunde, ein Implantat und ein Verfahren zur Herstellung eines Implantats anzugeben, so daß der Verschleiß bzw. Abrieb minimierbar oder zumindest verringerbar ist, daß die Bildung von Partikeln oder zumindest das Austreten von Partikeln verringerbar ist und/oder daß die Reibung verringerbar ist, insbesondere wobei die Standzeit der Lagerfläche oder einer sonstigen Oberfläche verlängerbar ist.

Die obige Aufgabe wird durch ein Implantat gemäß Anspruch 1 oder ein Verfahren gemäß Anspruch 12 gelöst. Vorteilhafte Weiterbildungen sind Gegenstand der Unteransprüche.

Eine grundlegende Idee der vorliegenden Erfindung liegt darin, die Lagerfläche des Implantats zumindest bereichsweise, vorzugsweise zumindest im gesamten Auflager- bzw. Belastungsbereich, mikro-rauh auszubilden, und zwar sehr einfach und kostengünstig durch Anätzen.

Unter "mikro-rauh" ist hier eine derart rauhe - vorzugsweise bis in den µm-Bereich reichende - Ausbildung der Oberfläche zu verstehen, daß Partikel, vorzugsweise von bis zu I µm oder sogar bis zu 10 µm, von Vertiefungen der Oberfläche zumindest teilweise aufgenommen und insbesondere darin eingelagert werden können.

Die mikro-rauhe Ausbildung der Lagerfläche führt zu mehreren Vorteilen:
Erstens, entstehende Partikel können in Vertiefungen aufgenommen und insbesondere dort dauerhaft eingelagert werden. Dies gilt insbesondere für sehr feine bzw. Mikro-Partikel, die primär beim Aufeinandergleiten von zwei Oberflächen entstehen. So kann die Drei-Körper-Abrasion wirksam verringert oder sogar minimiert werden.
Zweitens, die mikro-rauhe Lagerfläche kann sich leichter an eine zugeordnete Gegenlagerfläche anpassen. Dies wird insbesondere durch plastische Verformung bzw. Abplattung der Mikrohügel ermöglicht. So "läuft" sich das vorzugsweise als Gleitlager oder Gelenk ausgebildete Lager schneller ein.
Drittens, die Vertiefungen der mikro-rauhen Lageroberfläche können ein Schmiermittelreservoir bilden. Dies ist einer Verringerung der Reibung und/oder Erhöhung der Standzeit zuträglich.
Viertens, die mikro-rauhe Lagerfläche ist in ihrer Oberfläche gegenüber einer glatten Oberfläche wesentlich vergrößert. Die vergrößerte Oberfläche kann Partikel und/oder Schmiermittel besser binden bzw. festhalten. Dies ist wiederum einer Verringerung der Reibung und/oder Verlängerung der Standzeit, insbesondere durch Verringerung der durch freie Partikel verursachten Drei-Körper-Abrasion, zuträglich.

Vorzugsweise ist die mikro-rauhe Lagerfläche zumindest größtenteils, insbesondere insgesamt, makroskopisch glatt ausgebildet. Für das menschliche Auge erscheint die Lagerfläche also glatt, auch wenn ggf. Verfärbungen bzw. optische Effekte ein farblich variierendes Aussehen der Lageroberfläche bewirken können.

Bei einem Implantat mit einem derartigen Lager können durch die Aufnahme und Einlagerung der gerade beim anfänglichen Einlaufen des Lagers entstehenden Partikel die Abgabe von Partikeln und dementsprechend die potentielle Beeinträchtigung eines Patienten zumindest weitgehend vermieden oder zumindest minimiert werden. Der Einsatz des Lagers bei einem Implantat oder einer sonstigen Prothese stellt daher eine besonders bevorzugte Verwendung des vorschlagsgemäßen Lagers dar.

Gemäß einer besonders bevorzugten Ausführungsvariante sind die Vertiefungen der mikro-rauhen Lagerfläche zumindest teilweise mit einem Schmiermittel und/oder Kunststoff gefüllt. Hierdurch können optimale oder zumindest wesentliche bessere Trocken- und/oder Notlaufeigenschaften des Lagers erreicht werden. Insbesondere wird dabei eine wesentlich günstigere Herstellung ermöglicht, als dies beispielsweise bei den bekannten Sinter-Bronzen mit eingelagertem Schmiermittel der Fall ist.

Gemäß eine Weiterbildung der genannten Ausführungsvariante ist die mikro-rauhe Lagerfläche mit einer vorzugsweise zähelastischen Beschichtung versehen, die insbesondere in die Vertiefungen eingreift und/oder aus Kunststoff besteht. Aufgrund der mikro-rauhen Struktur und der sich durch das Anätzen vorzugsweise zusätzlich bildenden Feinstruktur bzw. Nanostruktur ist ein außerordentlich haltbarer Verbund aus mikro-rauher Oberfläche und Beschichtung erreichbar. Dieser Verbund ist als Lagerfläche bzw. Lagerteil einsetzbar, wobei die Beschichtung dann vorzugsweise aus einem Material mit zumindest ausreichenden Schmiereigenschaften gebildet ist.

Weitere Ziele, Vorteile, Eigenschaften und Merkmale der vorliegenden Erfindung werden nachfolgend anhand der Zeichnung näher erläutert. Es zeigt:
- Fig. 1: eine schematische Darstellung eines Lagers mit einer zumindest bereichsweise mikro-rauhen Lageroberfläche;
- Fig. 2: eine schematische, ausschnittsweise vergrößerte Schnittdarstellung der Lagerfläche unter Vernachlässigung der bei der Ausführungsform gemäß Fig. 1 vorhandenen Wölbung der Lagerfläche;
- Fig. 3: einen vergrößerten Ausschnitt von Fig. 2;
- Fig. 4: ein vorschlagsgemäßes, als Hüftgelenk ausgebildetes Implantat mit dem Lager; und
- Fig. 5a - 5c: schematische Schnittdarstellungen der Lagerfläche verschiedener Ausführungsvarianten.

In den Figuren werden für gleiche oder ähnliche Teile dieselben Bezugszeichen verwendet, wobei entsprechende oder vergleichbare Vorteile und Eigenschaften erreicht werden, auch wenn eine wiederholte Beschreibung aus Vereinfachungsgründen weggelassen ist.

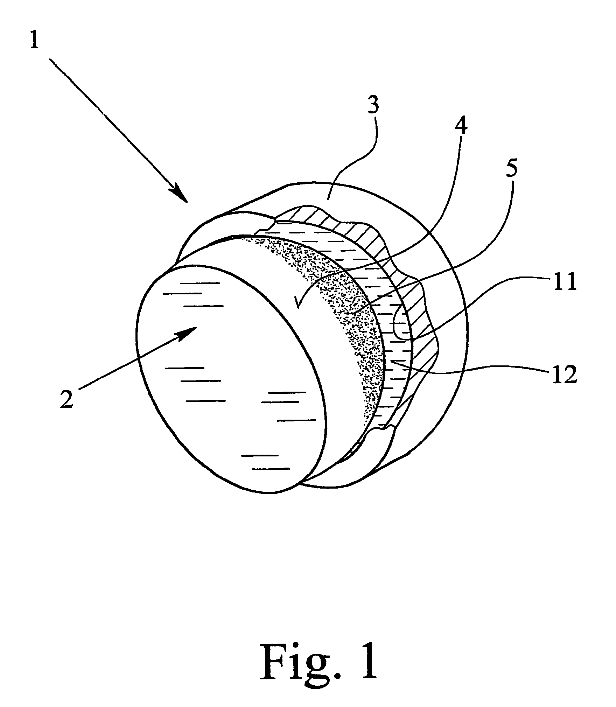

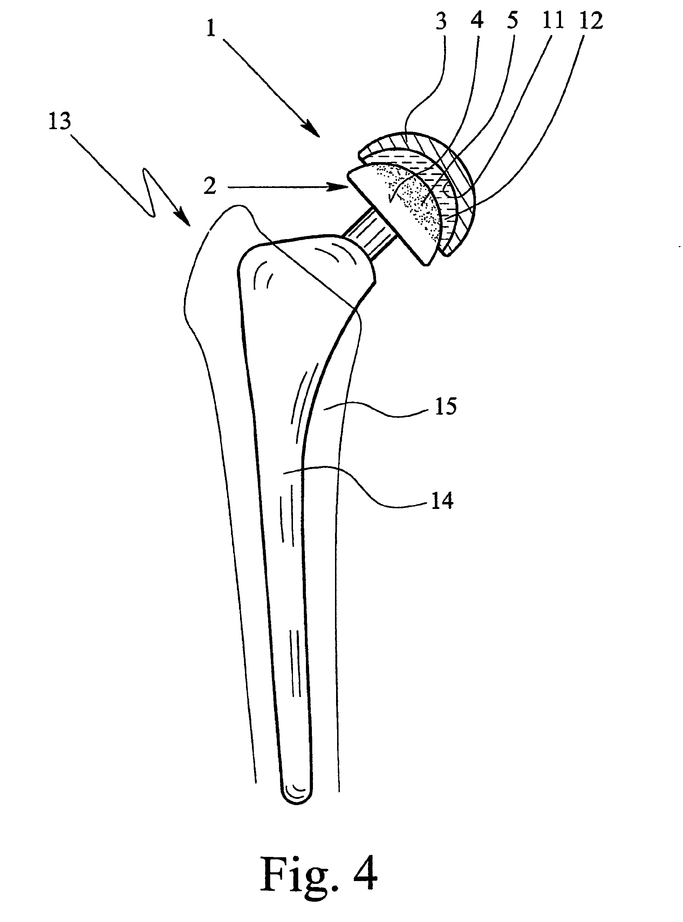

Fig. 1 zeigt in schematischer Darstellung ein Lager 1 eines in Fig. 4 dargestellten Implantats 13, wobei das Lager 1 beim Darstellungsbeispiel als Gleitlager ausgebildet ist. Jedoch kann es sich auch um ein sonstiges Lager, wie ein Rollen- oder Wälzlager, handeln.

Das dargestellte Lager 1 weist einen Lagerkopf 2 und eine zugeordnete Lagerschale 3 auf, die aus Veranschaulichungsgründen in einem voneinander abgerückten Zustand in Fig. 1 dargestellt sind. Zur Veranschaulichung ist die Lagerschale 3 außerdem im Schnitt dargestellt.

Anstelle der Ausbildung als Lagerkopf 2 und Lagerschale 3 können die einander zugeordneten Lagerelemente auch eine sonstige, an den jeweiligen Einsatzzweck angepaßte Form aufweisen. Insbesondere kann eine gleitende und/oder abrollende Lagerung vorgesehen sein.

Beim Darstellungsbeispiel weist das Lager 1 bzw. dessen Lagerkopf 2 eine vorzugsweise metallische Lagerfläche 4 auf, die zumindest in einem Bereich 5, insbesondere zumindest im gesamten Abroll- oder Auflagerbereich, mikro-rauh ausgebildet ist. Der mikro-rauhe Bereich 5 ist zur Veranschaulichung in Fig. 1 gepunktet dargestellt.

Tatsächlich ist die Aufrauhung der Lagerfläche 4 im Bereich 5 bzw. in der gesamten Lagerfläche 4 derart fein ausgebildet, daß die Lagerfläche 4 für das menschliche Auge optisch glatt erscheint, selbst wenn die Aufrauhung zu einem farblich unregelmäßigen Aussehen der Lagerfläche 4 im mikro-rauhen Bereich 5 führt.

Das Lager 1 ist aus einem geeigneten Material oder mehreren Materialien, wie Metall, Keramik, Kunststoff, Verbundwerkstoff o. dgl., hergestellt.

Die Lagerfläche 4 ist vorzugsweise aus einem zähen bzw. duktilen Material gebildet. Insbesondere ist die Lagerfläche 4 aus Kunststoff, Keramik oder Metall, vorzugsweise aus Stahl, Eisen, Titan, Chrom, einer auf Eisen, Titan oder Chrom basierenden Legierung und/oder einer Cobalt-Chrom-Legierung, gebildet.

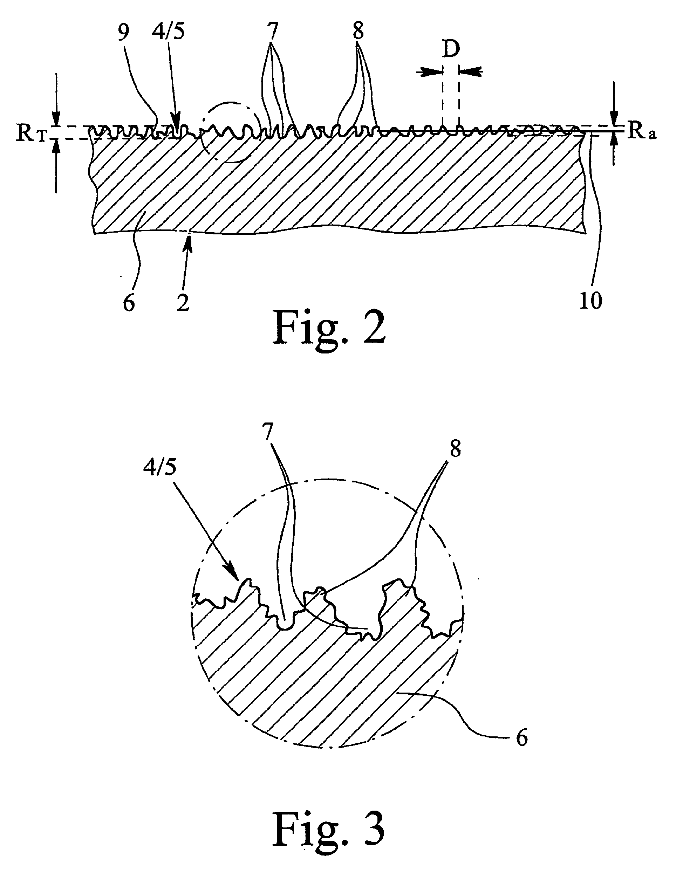

Fig. 2 zeigt in einer vergrößerten, ausschnittsweisen Schnittdarstellung die Lagerfläche 4 mit einer sich anschließenden Oberflächenschicht 6 des Lagerkopfs 2, wobei zur Vereinfachung der Darstellung die makroskopische Wölbung der Lagerfläche 4 beim Darstellungsbeispiel, also die kugelkopf- bzw. kalottenaratige Ausbildung der Lagerfläche 4 weggelassen wurde. Statt dessen ist die Lagerfläche 4 in Fig. 2 makroskopisch eben dargestellt.

Die mikro-rauhe Lagerfläche 4 ist zu ihrer Nanostrukturierung mit einer Vielzahl von Vertiefungen 7 und Erhebungen 8 versehen, die ineinander übergehen bzw. einander abwechseln. Insbesondere gehen die Vertiefungen 7 und Erhebungen 8 derart ineinander über, daß zumindest im wesentlichen keine planen Oberflächenabschnitte dazwischen gebildet werden.

Die tatsächliche Oberfläche 9 der mikro-rauhen Lagerfläche 4 ist dementsprechend deutlich größer als die durch die makroskopisch glatte Kontur 10 vermittelte makroskopische Erstreckungsfläche der Lagerfläche 4. Beim Darstellungsbeispiel beträgt die Oberfläche 9 vorzugsweise mindestens das 2-fache, insbesondere mindestens das 2,5-fache oder 3-fache, der makroskopischen Erstreckungsfläche der Lagerfläche 4.

Die in Fig. 2 lediglich auf der rechten Seite angedeutete, makroskopisch glatte Kontur 10 kann als das bei einer makroskopisch üblichen, beispielsweise spanenden oder schleifenden Bearbeitung gewünschte Sollprofil angesehen werden, das vorzugsweise makroskopisch glatt ist. Die Kontur 10 ist bei der Darstellung gemäß Fig. 2 zur Erläuterung bzw. Definition der mittleren Rauheit Rₐ nicht auf den Erhebungen 8, sondern zwischen den Erhebungen 8 und den Vertiefungen 7 eingezeichnet. Die mittlere Rauheit Rₐ stellt nämlich die mittlere Abweichung der Erhebungen 8 und Vertiefungen 7 von der mittleren, makroskopisch glatten Sollfläche bzw. Kontur 10 dar, wie in Fig. 2 angedeutet.

Die beiden gestrichelten Linien auf der rechten Seite von Fg. 2 deuten die Höhenabweichungen der Vertiefungen 7 bzw. der Erhebungen 8 von der mittleren, makroskopisch glatten Kontur 10 an. Aus diesen Abweichungen erfolgt die Bestimmung der genannten mittleren Rauheit Rₐ.

Vorzugsweise beträgt die mittlere Rauheit Rₐ der Lagerfläche 4 im mikro-rauhen Bereich 5 mindestens ein 1 nm, insbesondere mindestens 10 nm oder 100 nm, und/oder höchstens 10 µm, insbesondere bis zu 5 µm.

Die Rauhtiefe R_{T}, dh. die maximale Höhendifferenz zwischen einer der Erhebungen 8 und einer der Vertiefungen 7 im mikro-rauhen Bereich 5, beträgt vorzugsweise höchstens 20 µm, insbesondere maximal 10 µm.

Der mittlere Durchmesser D der Vertiefungen 7 beträgt vorzugsweise mindestens 1 µm, insbesondere mindestens 2 oder 5 µm und/oder vorzugsweise höchstens 50 µm, insbesondere bis zu höchstens 20 oder 10 µm. Ganz besonders bevorzugt ist ein Durchmesser D von 5 bis 20 µm.

Die Erhebungen 8 und die Vertiefungen 7 sind - je nach Anwendungsfall - mehr oder weniger unregelmäßig ausgebildet, wie in Fig. 2 schematisch angedeutet. Vorzugsweise sind die Erhebungen 8 und Vertiefungen 7 ihrerseits wieder auf ihrer Oberfläche strukturiert bzw. rauh ausgebildet, insbesondere nanostrukturiert, wie durch die ausschnittsweise schematische Vergrößerung gem. Fig. 3 angedeutet.

Jedoch ist grundsätzlich auch eine regelmäßige bzw. zumindest im wesentlichen gleichförmige Ausbildung der Vertiefungen 7 und/oder der Erhebungen 8 möglich.

Trotz der genannten Unregelmäßigkeit kann bei der mikro-rauhen Lagerfläche 4 von einer Profilierung bzw. Strukturierung im Mikro- oder Nanometerbereich, d.h. insbesondere mit Strukturbreiten von beispielsweise 100 nm bis 50 µm gesprochen werden. Die Strukturbreite bezeichnet hier das Maß, mit dem sich einzelne Strukturelemente, wie die Vertiefungen 7 oder Erhebungen 8, wiederholen, d.h. also beispielsweise den Mittenabstand voneinander benachbarter Erhebungen 8 oder voneinander benachbarter Vertiefungen 7.

Die Vertiefungen 7 bzw. Erhebungen 8 sind vorzugsweise unregelmäßig über die Lagerfläche 4 zumindest im mikro-rauhen Bereich 5 verteilt angeordnet, wobei die benachbarten Vertiefungen 7 durch vorzugsweise ebenfalls unregelmäßig geformte Erhebungen 8 voneinander getrennt sind. Grundsätzlich ist jedoch auch eine zumindest im wesentlichen gleichmäßige Verteilung der Vertiefungen 7 bzw. der Erhebungen 8 auf der Lagerfläche 4 möglich.

Die mittlere Flächendichte der Vertiefungen 7 bzw. Erhebungen 8 beträgt vorzugsweise mindestens 1·10⁵/mm², insbesondere mindestens 2·10⁵/mm² oder 5·10⁵/mm².

Der Lagerfläche 4 ist eine Gegenlagerfläche 11 zugeordnet, die beim Darstellungsbeispiel an der Lagerschale 3 ausgebildet ist, wie in Fig. 1 angedeutet. Die Gegenlagerfläche 11 ist beim Darstellungsbeispiel komplementär zur Lagerfläche 4 ausgebildet. Jedoch kann die Gegenlagerfläche 11 - je nach Verwendungszweck und Lageraufbau - auch eine von der komplementären Oberflächenform abweichende Form aufweisen. Dies gilt insbesondere für sonstige Gleitlager oder Rollen- bzw. Wälzlager.

Beim Darstellungsbeispiel gleiten die Lagerfläche 4 und die Gegenlagerfläche 11 aufeinander, bilden also ein Gleitlager. Jedoch können der Gleitbewegung auch abrollende Bewegungen überlagert sein. Wie bereits oben angesprochen, können grundsätzlich auch andere Lagerformen, beispielsweise mit ebener Lagerfläche 4 und/oder Gegenlagerfläche 11 oder mit primär abrollender Bewegung, realisiert werden.

Die Gegenlagerfläche 11 ist vorzugsweise zumindest im wesentlichen glatt, also vorzugsweise sowohl makroskopisch glatt als auch nanoskopisch glatt (d. h. nicht mikro-rauh) ausgebildet.

Bedarfsweise kann die Gegenlagerfläche 11 aber auch zumindest bereichsweise mikro-rauh ausgebildet werden. Gemäß einer Ausführungsvariante ist die Gegenlagerfläche 11 mit feinen nach außen hin offenen Poren bzw. Hohlräumen, beispielsweise mit einem mittleren Durchmesser von 100 nm bis 20 µm, versehen. Insbesondere ich dabei die Gegenlagerfläche 11 durch eine Oxidschicht aus einem sogenannten Ventilmetall (Bildung der Poren bzw. Hohlräume durch Anodisierung), vorzugsweise Aluminiumoxid, gebildet. Die Poren bzw. Hohlräume können dann zusätzlich entstehende Partikel aufnehmen und/oder als Schmiermittelreservoir dienen.

Die Gegenlagerfläche 11 ist aus einem geeigneten Material, wie Kunststoff, Keramik oder Metall, gebildet. Vorzugsweise ist die Gegenlagerfläche 11 härter als die Lagerfläche 4 bzw. dessen mikro-rauher Bereich 5, um die gewünschte Aufnahme von entstehenden Partikeln in den Vertiefungen 7 der Lagerfläche 4 zu erreichen. Insbesondere ist die Gegenlagerfläche 11 aus Siliciumdioxid oder Aluminiumoxid gebildet. Beispielsweise kann die Gegenlagerfläche 11 aber auch aus dem gleichen oder einem ähnlichen Material wie die Lagerfläche 4 gebildet sein.

Beim Darstellungsbeispiel ist die zumindest bereichsweise mikro-rauhe Lagerfläche 4 am Lagerkopf 2 und die Gegenlagerfläche 11 an der Lagerschale 3 ausgebildet. Dies kann jedoch auch umgekehrt sein.

Die Lagerfläche 4 und die Gegenlagerfläche 11 können je nach Einsatz unmittelbar aufeinander gleiten, also ggf. eine schmiermittelfreie Lagerung bilden. Vorzugsweise ist der Lagerfläche 4 zumindest im mikro-rauhen Bereich 5 ein Schmiermittel 12 zugeordnet, wie in Fig. 1 angedeutet.

Das vorschlagsgemäße Lager 1 wird vorzugsweise derart verwendet bzw. eingesetzt, daß die Flächenpressung der Lagerfläche 4 bzw. deren Bereich 5 höchstens 100 MPa, insbesondere höchstens 50 MPa oder 20 MPa, beträgt. Dies gilt insbesondere bei metallischer Ausbildung der Lagerfläche 4, hängt jedoch auch von dem verwendeten Material ab.

Die vorschlagsgemäße mikro-rauhe Ausbildung der Lagerfläche 4 führt insbesondere im Zusammenspiel mit einer vorzugsweise zumindest im wesentlichen glatten und/oder härteren Gegenlagerfläche 11 dazu, daß ein sehr schnelles Einlaufen bei geringer Partikelbildung oder zumindest geringer Partikelabscheidung ermöglicht wird. Zudem ergibt sich eine verhältnismäßig geringe Reibung. Dies läßt sich dadurch erklären, daß in der Einlaufphase eine schnelle Anpassung der vorzugsweise aus einem zähen und/oder duktilen Material, insbesondere Metall, gebildeten Lagerfläche 4 an die Gegenlagerfläche 11 erfolgt, wobei entstehende Partikel, die ansonsten zu der unerwünschten Drei-Körper-Abrasion führen können, von den Vertiefungen 7 der Lagerfläche 4 aufgenommen werden können. Zudem haftet das Schmiermittel 12 besonders gut auf der großen Oberfläche 9 der Lagerfläche 4, wobei sich außerdem ein verhältnismäßig großes Schmiermittelreservoir in den Vertiefungen 7 bildet, so daß eine geringe Reibung, insbesondere Gleitreibung, ermöglicht wird.

Versuche haben zudem gezeigt, daß ein weiterer vorteilhafter Effekt bei der vorschlagsgemäßen Lösung auftreten kann. Insbesondere bei metallischen Lagerflächen 4 können die entstehenden Metallpartikel - zumindest bereichsweise - auf den Erhebungen bzw. Mikrohügeln 8 eine sehr feste Partikelschicht von beispielsweise etwa 10 nm Dicke bilden. Die sich bildende Partikelschicht kann sich aufgrund der Vertiefungen 7 sehr gut mit der Lagerfläche 4 verbinden. Eine hohe Festigkeit der Partikelschicht kann sich insbesondere dadurch ergeben, daß die einzelnen Metallpartikel aufgrund ihrer geringen Größe zumindest teilweise, insbesondere zumindest weitgehend vollständig oxidieren. Es bildet sich dann eine besonders harte Schicht aus den zumindest teilweise oxidierten und/oder keramikartigen Partikeln, die dementsprechend sehr verschleißfest bzw. abriebfest ist.

Die Lagerfläche 4 wird durch Anätzen aufgerauht bzw. strukturiert, insbesondere durch Schwefelsäure und/oder Chromschwefelsäure. Dies gestattet eine einfache Herstellung. Beispielsweise wird die metallische, insbesondere aus rostfreiem Eisen bzw. Stahl oder einer Cobalt-Chrom-Legierung bestehende Lageroberfläche 4 der erwärmten Säure ausgesetzt. Bei einer Erwärmung der Säure auf etwa 200° C, etwa einmolaler Konzentration genügt beispielsweise eine Einwirkzeit von 30 Minuten bis 2 Stunden. Es erfolgt also eine naßchemische Behandlung bzw. Aufrauhung der Lageroberfläche 4.

Zusätzlich kann auch eine elektrochemische Unterstützung bzw. Förderung des Ätzprozesses erfolgen. Beispielsweise genügt dann etwa 30° C bis 70° C, vorzugsweise etwa 40° C warme Säure, um bei vergleichbarer Einwirkzeit die Lagerfläche 4 entsprechend anzuätzen, also partiell unter Bildung der Vertiefungen 7 abzutragen.

Fig. 4 zeigt in schematischer Darstellung das vorschlagsgemäße Implantat 13, das beim Darstellungsbeispiel als Gelenk, nämlich als Hüftgelenk, ausgebildet ist. Jedoch kann es sich beispielsweise auch um ein sonstiges Gelenk, wie ein künstliches Kniegelenk oder ein sonstiges, eine Lagerfunktion ausübendes Implantat oder eine sonstige Prothese mit einem Gelenk handeln.

Das dargestellte Implantat 13 bildet ein künstliches Hüftgelenk. Bei der Implantation wird ein Schaft 14 in einen in Fig. 4 angedeuteten Oberschenkelknochen 15 und die Lagerschale 3 in einen zugeordneten Hüftknochenbereich (nicht dargestellt) eingesetzt.

Das vorschlagsgemäße Implantat 13 bzw. dessen Lager 1 zeigt die bereits oben ausführlich erläuterten Vorteile. Insbesondere ist aus den genannten Gründen eine gegenüber herkömmlichen Implantaten wesentlich längere Standzeit erreichbar.

Vorzugsweise wird das Implantat 13 bzw. dessen Lager 1 derart verwendet bzw. eingesetzt, daß die Flächenpressung der Lagerfläche 4 bzw. dessen Bereich 5 höchstens 100 MPa, insbesondere höchstens 50 MPa oder 20 MPa, beträgt.

Vorzugsweise wird die Lageroberfläche 4 unmittelbar aus der Oberflächenschicht 6 bzw. dem Trägermaterial des Lagerkopfes 2 oder eines sonstigen Lagerteils gebildet. Eine zusätzliche Beschichtung zur Ausbildung der mikro-rauhen Lagerfläche 4 ist vorzugsweise also nicht vorgesehen und ermöglicht eine dementsprechend einfache und kostengünstige Herstellung.

Vorzugsweise ist vorgesehen, daß das Anätzen zur Aufrauhung bzw. Strukturierung der Lagerfläche 4 eine abschließende (formgebende oder mechanische) Bearbeitung der Lagerfläche 4 darstellt. Dies ist ebenfalls einer einfachen und damit kostengünstigen Herstellung zuträglich.

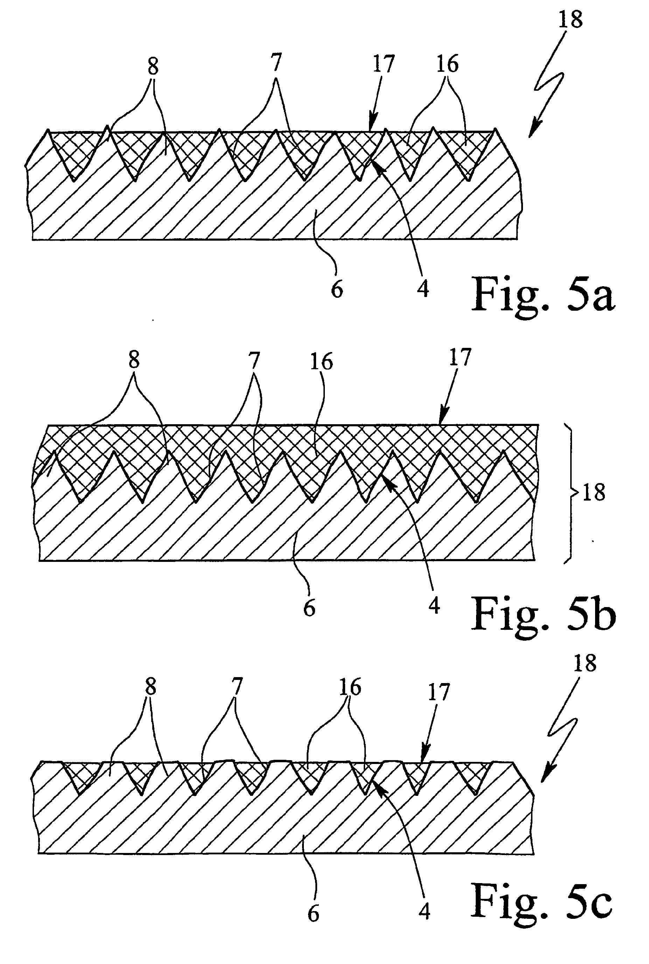

Fig. 5a bis c zeigen in schematischen Schnittdarstellungen die mikro-rauhe Lagerfläche 4 mit relativ gleichmäßiger bzw. regelmäßiger Strukturierung. Insbesondere sind die Vertiefungen 7 zumindest im wesentlichen kegelförmig und/oder die Erhebungen 8 zumindest im wesentlichen kegelförmig oder kegelstumpfförmig ausgebildet.

Die Vertiefungen 7 sind vorzugsweise zumindest teilweise mit einem Material 16 gefüllt. Insbesondere handelt es sich hierbei um ein Schmiermittel und/oder einen Kunststoff. Vorzugsweise ist das Material 16 weicher als die Deckschicht 6 bzw. ein sonstiges die Lagerfläche 4 bildendes Material 16 ausgebildet.

Das Material 16 bildet eine Beschichtung 17, die die Lageroberfläche 4 zumindest teilweise überdeckt. Bei der Ausführungsform gemäß Fig. 5b überdeckt die Beschichtung 17 die Lagerfläche 4 vollständig.

Im Fall der Ausführungsform gemäß Fig. 5c ist eine nur teilweise Überdeckung der Lagerfläche 4 bei kegelstumpfförmig ausgebildeten Erhebungen 8 vorgesehen. Hier schützen die Erhebungen 8 das in den Vertiefungen 7 befindliche Material 16 besonders gut gegen mechanische Einwirkungen.

Die Lagerfläche bildet mit dem Material 16 bzw. der Beschichtung 17 einen Verbundaufbau 18. Durch das Anätzen der insbesondere metallischen Lagerfläche 4 wird ein ausgezeichneter Verbund mit dem Material 16, insbesondere Kunststoff, ermöglicht. Folglich ist der Verbundaufbau 18 ausgezeichnet haltbar; die Beschichtung 17 haftet also ausgezeichnet auf der Lagerfläche 4.

Der Verbundaufbau 18 ist vorzugsweise für hochbelastete Werkstücke, insbesondere Lager oder dergleichen, vorgesehen. Jedoch kann der Verbundaufbau 18 auch für sonstige Zwecke, insbesondere in der bevorzugten Kombination von Metall-Oberfläche 4 und Kunststoff-Beschichtung 17, eingesetzt werden.

Je nach Einsatzzweck handelt es sich bei dem Material 16 insbesondere um PTFE (Polytetraflurethylene), PFA (Perlfluor-Alkoxy-Polymere), PEEK (Polyetheretherkethon) oder sonstige geeignete Kunststoffe, wie insbesondere in der Veröffentlichung "Friction and wear of highly stressed thermoplastics bearings under dry sliding conditions" von S. Marx, R. Junghaus in Wear 193 (1996) 253-260 genannt, deren gesamter Inhalt hiermit vollinhaltlich ergänzend als Offenbarung eingeführt wird.

Wie bereits angedeutet, kann das Material 16 bei entsprechender Auswahl insbesondere auch als Schmiermittel wirken, um bestimmte Trockenlauf- und/oder Notlaufeigenschaften bei Einsatz des vorschlagsgemäßen Verbundaufbaus 18 im Lager 1 zu erreichen.

## Patentansprüche

1. Implantat (13), insbesondere Gelenk, wie Hüftgelenk oder Kniegelenk, mit einem Gleitlager (1) mit einer durch Vertiefungen (7) mikro-rauhen Lagerfläche (4), wobei die Vertiefungen (7) durch Anätzen der Lagerfläche (4) gebildet sind.

2. Implantat nach Anspruch 1, **dadurch gekennzeichnet, daß** die Lagerfläche (4) makroskopisch glatt ausgebildet ist und/oder durch das Anätzen abschließend (formgebend oder mechanisch) bearbeitet ist.

3. Implantat nach Anspruch 1 oder 2, **dadurch gekennzeichnet, daß** die Lagerfläche (4) aus einem zähen Material und/oder aus Kunststoff, Keramik oder Metall, insbesondere aus Stahl, Eisen, Titan, Chrom, einer auf Eisen, Titan oder Chrom basierenden Legierung und/oder einer Cobalt-Chrom-Legierung, gebildet ist.

4. Implantat nach einem der voranstehenden Ansprüche, **dadurch gekennzeichnet, daß** die Lagerfläche (4) eine mittlere Rauheit (Rₐ) und/oder eine Rauhtiefe (R_{T}) von höchstens 20 µm, insbesondere bis zu 10 µm oder 5 µm, aufweist.

5. Implantat nach einem der voranstehenden Ansprüche, **dadurch gekennzeichnet, daß** die Lagerfläche (4) zwischen den Vertiefungen (7) zumindest im wesentlichen keine ebenen Oberflächenabschnitte aufweist.

6. Implantat nach einem der voranstehenden Ansprüche, **dadurch gekennzeichnet, daß** die Vertiefungen (7) einen mittleren Durchmesser (D) von 1 bis 50 µm, vorzugsweise von 5 bis 20 µm aufweisen, und/oder die Vertiefungen (7) unregelmäßig verteilt angeordnet sind.

7. Implantat nach einem der voranstehenden Ansprüche, **dadurch gekennzeichnet, daß** die Lagerfläche (4) mit den Vertiefungen (7) mit einer Flächendichte von mindestens 1·10⁵/mm², insbesondere mindestens 2·10⁵/mm² oder 5·10⁵/mm², versehen ist.

8. Implantat nach einem der voranstehenden Ansprüche, **dadurch gekennzeichnet, daß** der Lagerfläche (4) eine Gegenlagerfläche (11) zugeordnet ist, die härter als die Lagerfläche (4) ist.

9. Implantat nach einem der voranstehenden Ansprüche, **dadurch gekennzeichnet, daß** die Vertiefungen (7) zumindest im wesentlichen kegelförmig ausgebildet sind und/oder daß Erhebungen (8) zwischen den Vertiefungen (7) zumindest im wesentlichen kegelförmig oder kegelstumpfförmig ausgebildet sind.

10. Implantat nach einem der voranstehenden Ansprüche, **dadurch gekennzeichnet, daß** die Vertiefungen (7) zumindest teilweise mit einem Schmiermittel, insbesondere Kunststoff (16), vorzugsweise PTFE, PFA oder PEEK, gefüllt sind.

11. Implantat nach einem der voranstehenden Ansprüche, **dadurch gekennzeichnet, daß** die Lagerfläche (4) mit einer vorzugsweise zähelastischen Beschichtung (17), insbesondere aus Kunststoff (16), vorzugsweise PTFE, PFA oder PEEK, versehen ist.

12. Verfahren zur Herstellung eines Implantats (13) mit einer mikro-rauhen Lagerfläche (4), wobei daß die Fläche (4) naßchemisch durch Anätzen aufgerauht wird und durch das Anätzen unregelmäßig verteilt angeordnete Vertiefungen (7) gebildet werden, wobei die Vertiefungen (7) zumindest im wesentlichen kegelförmig und/oder Erhebungen (8) zwischen den Vertiefungen (7) zumindest im wesentlichen kegelförmig oder kegelstumpfförmig ausgebildet werden.

13. Verfahren nach Anspruch 12, **dadurch gekennzeichnet, daß** die Fläche (4) aus Metall, Keramik oder Kunststoff, insbesondere aus Stahl, Eisen, Titan, Chrom, einer auf Eisen, Titan oder Chrom basierenden Legierung und/oder einer Cobalt-Chrom-Legierung, gebildet wird und/oder elektrochemisch angeätzt wird.

14. Verfahren nach Anspruch 12 oder 13, **dadurch gekennzeichnet, daß** das Anätzen durch die Einwirkung von insbesondere erwärmter Säure, vorzugsweise von Chromschwefelsäure und/oder Schwefelsäure, erfolgt und/oder eine abschließende (formgebende oder mechanische) Bearbeitung der Fläche (4) darstellt.

## Claims

1. Implant (13), in particular a joint, such as a hip joint or knee joint, with a sliding bearing (1), with a bearing surface (4) made micro-rough by depressions (7), the depressions (7) being formed by etching of the bearing surface (4).

2. Implant according to claim 1, **characterized in that** the bearing surface (4) is made to have a macroscopically smooth form and/or is finally (shapingly or mechanically) machined by the etching.

3. Implant according to claim 1 or 2, **characterized in that** the bearing surface (4) is formed from a tough material and/or from plastic, ceramic or metal, in particular from steel, iron, titanium, chromium, an alloy based on iron, titanium or chromium and/or a cobalt-chromium alloy.

4. Implant according to any one of the preceding claims, **characterized in that** the bearing surface (4) has an averaged roughness (Rₐ) and/or a peak-to-valley height (R_{T}) of at most 20 µm, in particular up to 10 µm or 5 µm.

5. Implant according to any one of the preceding claims, **characterized in that** the bearing surface (4) has at least substantially no planar surface portions between the depressions (7).

6. Implant according to any one of the preceding claims, **characterized in that** the depressions (7) have an average diameter (D) of from I to 50 µm, preferably from 5 to 20 µm, and/or the depressions (7) are arranged in an irregular distribution.

7. Implant according to any one of the preceding claims, **characterized in that** the bearing surface (4) is provided with the depressions (7) with a surface density of at least 1·10⁵/mm², in particular at least 2·10⁵/mm² or 5·10⁵/mm².

8. Implant according to any one of the preceding claims, **characterized in that** the bearing surface (4) is assigned a counterbearing surface (11), which is harder than the bearing surface (4).

9. Implant according to any one of the preceding claims, **characterized in that** the depressions (7) are formed at least substantially conically and/or **in that** elevations (8) between the depressions (7) are formed at least substantially conically or frustoconically.

10. Implant according to any one of the preceding claims, **characterized in that** the depressions (7) are at least partially filled with a lubricant, in particular plastic (16), preferably PTFE, PFA or PEEK.

11. Implant according to any one of the preceding claims, **characterized in that** the bearing surface (4) is provided with a preferably viscoelastic coating (17), in particular made of plastic (16), preferably PTFE, PFA or PEEK.

12. Method for producing an implant (13) with a micro-rough bearing surface (4), wherein the surface (4) is roughened wet-chemically by etching and depressions (7) are formed by the etching in an irregular distribution, wherein the depressions (7) are formed at least substantially conically and/or elevations (8) between the depressions (7) are formed at least substantially conically or frustoconically.

13. Method according to claim 18, **characterized in that** the surface (4) is formed from metal, ceramic or plastic, in particular from steel, iron, titanium, chromium, an alloy based on iron, titanium or chromium and/or a cobalt-chromium alloy, and/or is electrochemically etched.

14. Method according to claim 12 or 13, **characterized in that** the etching is performed by exposure to acid, in particular heated acid, preferably chromosulfuric acid and/or sulfuric acid, and/or represents a final (shaping or mechanical) machining of the surface (4)..

## Revendications

1. Implant (13), en particulier articulation telle qu'une articulation de la hanche ou une articulation du genou, comprenant un palier à glissement (1) muni d'une surface d'appui (4) comportant des microrugosités obtenues via des renfoncements (7), les renfoncements (7) étant réalisés par attaque chimique naissante de la surface d'appui (4).

2. Implant selon la revendication 1, **caractérisé en ce que** la surface d'appui (4) est réalisée de telle sorte qu'elle possède un lissé macroscopique et/ou est soumise à un traitement ultérieur (de façonnement ou de type mécanique), via l'attaque chimique naissante.

3. Implant selon la revendication 1 ou 2, **caractérisé en ce que** la surface d'appui (4) est constituée d'une matière ductile et/ou d'une matière synthétique, d'une matière céramique ou d'un métal, en particulier d'acier, de fer, de titane, de chrome, d'un alliage à base de fer, de titane ou de chrome et/ou d'un alliage de cobalt-chrome.

4. Implant selon l'une quelconque des revendications précédentes, **caractérisé en ce que** la surface d'appui (4) présente une rugosité moyenne (Rₐ) et/ou une profondeur de rugosité (R_{T}) maximales de 20 µm, en particulier jusqu'à 10 µm ou 5 µm.

5. Implant selon l'une quelconque des revendications précédentes, **caractérisé en ce que** la surface d'appui (4) ne présente, entre les renfoncements (7), au moins à titre essentiel, aucune section superficielle plane.

6. Implant selon l'une quelconque des revendications précédentes, **caractérisé en ce que** les renfoncements (7) présentent un diamètre moyen (D) de 1 à 50 µm, de préférence de 5 à 20 µm, et/ou les renfoncements (7) sont disposés selon une répartition irrégulière.

7. Implant selon l'une quelconque des revendications précédentes, **caractérisé en ce que** la surface d'appui (4) comprenant les renfoncements (7) est munie d'une densité superficielle d'au moins 1·10⁵/mm², en particulier d'au moins 2·10⁵/mm² ou 5·10⁵/mm².

8. Implant selon l'une quelconque des revendications précédentes, **caractérisé en ce qu'**une surface de butée (11) est attribuée à la surface d'appui (4), qui est plus dure que la surface d'appui (4).

9. Implant selon l'une quelconque des revendications précédentes, **caractérisé en ce que** les renfoncements (7) sont réalisés pour prendre au moins essentiellement une forme sphérique et/ou **en ce qu'**on prévoit des élévations (8) entre les renfoncements (7), au moins essentiellement de forme sphérique ou de forme tronconique.

10. Implant selon l'une quelconque des revendications précédentes, **caractérisé en ce que** les renfoncements (7) sont remplis, au moins en partie, avec un lubrifiant, en particulier une matière synthétique (16), de préférence du PTFE, du PFA ou du PEEK.

11. Implant selon l'une quelconque des revendications précédentes, **caractérisé en ce que** la surface d'appui (4) est munie d'un revêtement de préférence viscoélastique (17), en particulier constitué d'une matière synthétique (16), de préférence du PTFE, du PFA ou du PEEK.

12. Procédé pour la fabrication d'un implant (13) comprenant une surface d'appui microrugueuse (4), dans lequel la surface (4) est rendue rugueuse par attaque chimique naissante dans un procédé au mouillé et dans lequel on obtient, grâce à l'attaque chimique naissante, la formation de renfoncements (7) disposés selon une répartition irrégulière, les renfoncements (7) étant réalisés au moins à titre essentiel pour prendre une forme sphérique et/ou des élévations (8), entre les renfoncements (7), étant réalisées pour prendre au moins essentiellement une forme sphérique ou une forme tronconique.

13. Procédé selon la revendication 12, **caractérisé en ce que** la surface (4) est réalisée à partir d'un métal, d'une matière céramique ou d'une matière synthétique, en particulier d'acier, de fer, de titane, de chrome, d'un alliage à base de fer, de titane ou de chrome et/ou d'un alliage de cobalt-chrome et/ou est soumise à une attaque naissante par voie électrochimique.

14. Procédé selon la revendication 12 ou 13, **caractérisé en ce que** l'attaque chimique naissante a lieu en laissant agir de l'acide, en particulier de l'acide réchauffé, de préférence de l'acide chromosulfurique et/ou de l'acide sulfurique et/ou représente un traitement ultérieur (par façonnement ou de type mécanique) de la surface (4).
